(19) Europäisches Patentamt / European Patent Office / Office européen des brevets

(11) **EP 2 666 869 B1**

(12) **EUROPEAN PATENT SPECIFICATION**

(45) Date of publication and mention of the grant of the patent:
**10.12.2014 Bulletin 2014/50**

(51) Int Cl.:
*C12Q 1/68* (2006.01)

(21) Application number: **12169495.4**

(22) Date of filing: **25.05.2012**

(54) **Method for determining the risk of toxicity of sorafenib in cancer patients**

Verfahren zur Bestimmung der Toxizitätsgefahr von Sorafenib bei Krebspatienten

Procédé permettant de déterminer le risque de toxicité de Sorafenib chez des patients atteints de cancer

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.11.2013 Bulletin 2013/48**

(73) Proprietor: **Université Paris Descartes**
**75006 Paris (FR)**

(72) Inventors:
• **Blanchet, Benoît**
**75011 Paris (FR)**
• **Narjoz, Céline**
**75020 Paris (FR)**
• **Boudou-Rouquette, Pascaline**
**75012 Paris (FR)**
• **Loriot, Marie-Anne**
**92230 Gennevilliers (FR)**

(74) Representative: **Cabinet Plasseraud**
**52, rue de la Victoire**
**75440 Paris Cedex 09 (FR)**

(56) References cited:
• **SHIM J H ET AL: "GENETIC PREDISPOSITION OF HAND-FOOT SKIN REACTION FOLLOWING SORAFENIB THERAPY IN KOREAN PATIENTS WITH HEPATOCELLULAR CARCINOMA", JOURNAL OF HEPATOLOGY, vol. 54, no. Suppl. 1, March 2011 (2011-03), page S264, XP009162285, & 46TH ANNUAL MEETING OF THE EUROPEAN-ASSOCIATION-FOR-THE-STUDY-OF-THE- LIVER (EASL); BERLIN, GERMANY; MARCH 30 -APRIL 03, 2011 ISSN: 0168-8278**
• **LOKESH JAIN ET AL: "Population pharmacokinetic analysis of sorafenib in patients with solid tumours", BRITISH JOURNAL OF CLINICAL PHARMACOLOGY, vol. 72, no. 2, 1 August 2011 (2011-08-01) , pages 294-305, XP55036366, ISSN: 0306-5251, DOI: 10.1111/j. 1365-2125.2011.03963.x**
• **C. J. PEER ET AL: "Sorafenib Is an Inhibitor of UGT1A1 but Is Metabolized by UGT1A9: Implications of Genetic Variants on Pharmacokinetics and Hyperbilirubinemia", CLINICAL CANCER RESEARCH, vol. 18, no. 7, 1 April 2012 (2012-04-01), pages 2099-2107, XP55036364, ISSN: 1078-0432, DOI: 10.1158/1078-0432.CCR-11-2484**
• **"Sorafenib", Wikipedia , January 2012 (2012-01), XP007920968, Retrieved from the Internet: URL: http://en.wikipedia.org/wiki/Sorafenib [retrieved on 2012-08-28]**

**Description**

BACKGROUND OF THE INVENTION

Sorafenib is used to treat advanced renal cell carcinoma (a type of cancer that begins in the kidneys) and is also used to treat unresectable hepatocellular carcinoma (a type of liver cancer that cannot be treated with surgery). It is also a promising treatment for kidney, thyroid, lung or brain cancer. Sorafenib belongs to a class of medications called multikinase inhibitors. It works by slowing the spread of cancer cells. As a dual-action inhibitor, it targets RAF/MEK/ERK pathway in tumor cells and tyrosine kinases VEGFR/PDGFR in tumor vasculature [1].

Meanwhile, several side effects have been observed in some patients upon sorafenib intake. Notably, hand-foot skin reaction (HFSR), diarrhea, asthenia and hypertension are the most frequent adverse reactions of clinical importance in sorafenib-treated patients [3]. These toxicities are often manageable with ancillary treatments. However they may lead to drug discontinuation or dose reduction that can decrease the potential life-prolonging benefits of sorafenib [4]. Moreover, each person's reaction to sorafenib treatment is different. Some people have very few side effects while others may experience more. Due to the widespread use of sorafenib and the associated risk of side effects, the question of defining subgroups of patients susceptible to sorafenib-related toxicities is of crucial clinical importance. Identifying predictive biomarkers of sorafenib response is not only crucial to prognose the individuals susceptible to sorafenib toxicity before such treatment, but also helpful in improving therapy management or drug dose determination during cancer therapy based on the overall clinical situation.

However, data regarding determinants of sorafenib-induced toxicity still remain scarce. Existing indexes include age for therapy discontinuation in Japanese patients [30] and cumulative sorafenib dose, ECOG (Eastern Cooperative Oncology Group) performance status and female gender as determinants for risk of HFSR [31,32]. These determinants are either not practical or not specific enough (such as age and gender). Additionally, no efficient index has yet been found for predicting the risk of diarrhea.

When looking into sorafenib's metabolic pathways for new possibilities of index, it is understood that sorafenib is metabolized primarily in the liver and undergoes oxidative metabolism mediated by cytochrome P450 3A4 isoform (CYP3A4), as well as glucuronidation mediated by uridine diphosphate glucuronyl transferase 1A9 (*UGT1A9*) [5].

So far, as regards these pharmacogenetic variants, an investigation has evaluated the effect of genotype with respect to CYP3A4*1B, CYP3A5*3C, UGT1A9*3 and UGT1A9*5 on sorafenib pharmacokinetics [8]. None of these pharmacogenetic variants was associated with sorafenib pharmacokinetics in the result of the said investigation.

An association between SNPs UGT1A9 IVS1-37877TT and UGT1A9 IVS1-37431AA with sorafenib-induced hand-foot skin reaction in the course of the treatment of hepatocellular carcinoma has been described [43].

Thus, there is still an unmet need for methods for predicting the risk of toxicity of sorafenib in patient.

SUMMARY OF THE INVENTION

The present inventors have surprisingly discovered that a single nucleotide polymorphism (SNP) within the *UGT1A9* gene is associated with an increased risk of toxicity of sorafenib in an individual upon sorafenib intake. Therefore, this SNP is useful for methods of determining the risk of toxicity of sorafenib, to identify patients at higher risk before treatment or to support an a priori dose reduction during treatment.

Accordingly, one aspect disclosed herein provides an in vitro method for determining the risk of toxicity of sorafenib in an individual upon sorafenib intake, comprising analyzing the nucleotide sequence of the *UGT1A9* gene for the presence of the single nucleotide polymorphism (SNP) consisting of *UGT1A9* -2152 C>T (rs 17868320), in a biological sample obtained from said individual.

Further scope of the applicability of the present invention will become apparent from the detailed description and drawings provided below. It should be understood, however, that the following detailed description and examples, while indicating embodiments of the invention, are given by way of illustration only, since various changes and modifications of the invention will become apparent to those skilled in the art from the following detailed description.

DETAILED DESCRIPTION OF THE EMBODIMENTS

The invention is defined in the appendant claims.

One aspect disclosed herein provides an in vitro method for determining the risk of toxicity of sorafenib in an individual upon sorafenib intake, comprising analyzing the nucleotide sequence of the gene *UGT1A9* for the presence of the single nucleotide polymorphism (SNP) consisting of *UGT1A9* -2152 C>T, in a biological sample obtained from said individual.

*UGT1A9* is a gene belonging to Homo sapiens UDP glucuronosyltransferase 1 family encoding a UDP-glucuronosyltransferase, which is an enzyme of the glucuronidation pathway that transforms small lipophilic molecules, such as steroids, bilirubin, hormones, and drugs, into water-soluble, excretable metabolites. This gene is part of a complex locus that encodes several UDP-glucuronosyltransferases.

The *UGT1A9* protein plays a central role in the metabolism of various classes of therapeutic drugs in addition to carcinogens and steroids. The great inter-individual variability of *UGT1A9*-mediated glucuronidation remains poorly explained.

The term "allele" used herein is one of two or more forms of a gene or a genetic locus (generally a group of genes). Most multicellular organisms have two sets of chromosomes, that is, they are diploid. These chromosomes are referred to as homologous chromosomes. Diploid organisms have one copy of each gene (and therefore one allele) on each chromosome. If both alleles are the same, they are homozygotes. If the alleles are different, they are heterozygotes.

Herein the allele nomenclature used for *UGT1A9* alleles and Single Nucleotide Polymorphisms is based primarily on the standard nomenclature for this gene, as described on the website:http://www.pharmacogenomics.pha.ulaval.ca/files/conten t/sites/pharmacogenomics/files/Nomenclature/UGT1A/UGT1A9.htm, partially reproduced in the Table below.

**Table: UGT1A9 allele nomenclature [41]**

| Allele naming | Protein | Nucleotide change | Genbank | References | Notes |
|---|---|---|---|---|---|
| UGT1A9*1a | UGT1A9.1 | | AC019072; AF297093 | | |
| UGT1A9*1b | UGT1A9.1 | -118 (dT)$_{9>10}$ | N/A | Yamanaka et al. 2004 | Originally named UGT1A9*22 |
| UGT1A9*1c | UGT1A9.1 | -440(T>C)/-331(C>T) | N/A | Girard et al. 2004 | |
| UGT1A9*1d | UGT1A9.1 | -440(T>C)/-331(C>T)/-118(dT)$_{9>10}$ | N/A | Girard et al. 2004 | |
| UGT1A9*1e | UGT1A9.1 | -87(G>A) | N/A | Girard et al. 2004 | |
| UGT1A9*1f | UGT1A9.1 | -1818(T>C)/-440(T>C)/- 331(C>T)/-118(dT)$_{9>10}$ | N/A | Girard et al. 2004 | |
| UGT1A9*1g | UGT1A9.1 | -1887(T>G)/ -440(T>C)/-331(C>T) | N/A | Girard et al. 2004 | |
| UGT1A9*1h | UGT1A9.1 | -1887(T>G)/-665(C>T)/-440(T>C)/-331(C>T) | N/A | Girard et al. 2004 | |
| UGT1A9*1j | UGT1A9.1 | -1818(T>C)/-440(T>C)/-331(C>T) | N/A | Girard et al. 2004 | |
| UGT1A9*1k | UGT1A9.1 | -1818(T>C)/-665(C>T)/-440(T>C)/-331(C>T)/-118(dT)$_{9>10}$ | N/A | Girard et al. 2004 | |
| UGTIA9*11 | UGT1A9.1 | -2152(C>T)/-440(T>C)/-331(C>T)/-275(T>A) | N/A | Girard et al. 2004 | |
| UGT1A9*1m | UGT1A9.1 | -665(C>T)/-440(T>C)/-331(C>T)/-118(dT)$_{9>10}$ | N/A | Girard et al. 2004 | |
| UGT1A9*1n | UGT1A9.1 | -2152(C>T)/-665(C>T)/-440(T>C)/-331(C>T)/-275(T>A) | N/A | Girard et al. 2004 | |
| UGT1A9*1p | UGT1A9.1 | -1818(T>C)/-665(C>T)/-440(T>C)/-331(C>T) | N/A | Girard et al. 2004 | |

| Allele naming | Protein | Nucleotide change | Genbank | References | Notes |
|---|---|---|---|---|---|
| UGT1A9*1q | UGT1A9.1 | I219 (A>T)/I313 (C>A)/I399 (C>T) | | Carlini et al. 2005 Girard et al. 2006 | |
| UGT1A9*1r | UGT1A9.1 | I152 (G>A)/I219 (A>T)/I313 (C>A) | | Carlini et al. 2005 | |
| UGT1A9*1s | UGT1A9.1 | -87 (G>A)/I219 (A>T)/I313 (C>A) | | Carlini et al. 2005 | |
| UGT1A9*1t | UGT1A9.1 | I143 (C>T)/I152 (G>A)/I219 (A>T)/I313 (C>A) | | Carlini et al. 2005 | |
| UGT1A9*1u | UGT1A9.1 | -118(dT)$_{9>10}$/I219 (A>T)/I313 (C>A)/I399 (C>T) | | Girard et al. 2006 | |
| UGT1A9*1v | UGT1A9.1 | -5366 (G>T)/ -4549 (T>C)/-2152(C>T)/-275(T>A)/ I219 (A>T)/I313 (C>A)/I399 (C>T) | | Girard et al. 2006 | |
| UGT1A9*1w | UGT1A9.1 | -5366 (G>T)/ -4549 (T>C)/-2152(C>T)/-275(T>A) | | Girard et al. 2006 | |
| UGT1A9*1x | UGT1A9.1 | I190 (T>C) | | Fujuta et al. 2006 | |
| UGT1A9*2 | UGT1A9.2 | 8 (G>A) | AF481811 | Villeneuve et al. 2003 | |
| UGT1A9*3a | UGT1A9.3 | 98(T>C) | AF481810 | Villeneuve et al. 2003 | |
| UGT1A9*3b | UGT1A9.3 | -1818(T>C)/-440(T>C)/- 331(C>T)/98(T>C) | N/A | Girard et al. 2004 | |
| UGT1A9*4 | UGT1A9.4 | 726 (T>G) | N/A | Saeki et al., 2003 | |
| UGT1A9*5 | UGT1A9.5 | 766 (G>A) | N/A | Jinno et al. 2003 | |

A single-nucleotide polymorphism (SNP) is a DNA sequence variation occurring when a single nucleotide - A, T, C or G - in the genome (or other shared sequence) differs between members of a biological species or paired chromosomes in an individual. For example, *UGT1A9* -2152 C>T means the nucleotide at position -2152 of the UGT gene is replaced by T instead of C. This SNP is located within the promoter region of the *UGT1A9* gene and is also identified by serial number rs 17868320. These genetic variations underlie differences in susceptibility to, or protection from all kinds of diseases. The severity of illness and the way the body responds to treatments are also manifestations of genetic variations.

The serial numbers or refSNP cluster ID numbers as used herein are based on the Single Nucleotide Polymorphism Database (dbSNP), which is a free public archive for genetic variation within and across different species developed and hosted by the National Center for Biotechnology Information (NCBI) in collaboration with the National Human Genome Research Institute (NHGRI).

Typically, the presence of the Single Nucleotide Polymorphism (SNP) *UGT1A9* -2152 C>T is indicative of a greater risk of toxicity upon sorafenib intake.

In one embodiment, the presence of *UGT1A9* -2152 C>T in an individual of female gender is indicative of an even greater risk of toxicity upon sorafenib intake.

In one embodiment, the *UGT1A9* gene further displays at least one SNP selected from the group consisting of -440 T>C, -331 C>T, -275 T>A, -665 C>T, -5366 G>T, -4549 T>C, I219 A>T, I313 C>A and I399 C>T.

In one embodiment, said *UGT1A9* gene comprises one or both alleles consisting of *UGT1A9*1L allele, i.e. one or both two alleles are the *UGT1A9* allele comprising the following SNPs - 2152 C>T / -440 T>C / -331 C>T / -275 T>A.

In another embodiment, said *UGT1A9* gene comprises one of both alleles consisting of *UGT1A9*1N allele, i.e one or both two alleles are the *UGT1A9* allele comprising the following SNPs -2152 C>T / -665 C>T / -440 T>C / -331 C>T / -275 T>A.

In yet another embodiment, said *UGT1A9* gene consists of heterozygous alleles consisting of the combination of *UGT1A9*1L and *UGT1A9*1N.

As used herein, the term "toxicity" has its general meaning in the art. It refers to any adverse drug reactions (ADR) defined by World Health Organization, namely a response to a medicine which is noxious and unintended, and which occurs at doses normally used in man.

Typically, the term "toxicity" as used herein can include, but is not limited to, the commonly observed side effects associated with sorafenib intake, such as Hand-foot skin reaction (HFSR), diarrhea, asthenia, hypertension or combination thereof.

In one embodiment, said risk is suffering from diarrhea. And it is further preferred that the severity grade of said diarrhea is at least 2 according to the NCI-Common Terminology Criteria for Adverse Events scale (NCI-CTCAE) 3.0 grading version.

In one embodiment, said individual is a human being and more preferably, said individual is a human patient suffering from cancer.

As used herein, the term "cancer" has its general meaning in the art. It refers to a disease in which abnormal cells divide without control and are able to invade other tissues. Cancer cells can spread to other parts of the body through the blood and lymph systems.

Typically, said patient is afflicted with renal, liver, kidney, thyroid, lung or brain cancer. More specifically, said renal cancer can be advanced renal cell carcinoma, and said liver cancer can be unresectable hepatocellular carcinoma.

In yet another embodiment, the said patient is afflicted with an advanced or metastatic solid tumor.

The nucleotide sequence is generally obtained from a genomic DNA sample isolated from the biological sample.

For the assay of genomic DNA, virtually any biological sample containing genomic DNA (e.g. not pure red blood cells) can be used. For example, and without limitation to, genomic DNA can be conveniently obtained from blood, semen, saliva, tears, urine, fecal material, sweat, buccal cells, skin, hair or other tissue containing nucleic acid of the individual.

It is more preferred that the sample contains peripheral blood leukocytes.

The method of invention is illustrated in the examples. The results highlight that the risk of sorafenib-induced grade $\geq$ 2 diarrhea strongly increases when the T allele in *UGT1A9* -2152 C>T is present (OR 14.33). The risk of toxicity further increases when the individual is of female gender. Therefore, those possessing the SNP of *UGT1A9* -2152 C>T are not suitable to be treated with sorafenib or need a dose reduction.

In one embodiment of the invention, the method is performed in a patient before sorafenib is prescribed. The results have shown that *UGT1A9* genotyping before treatment initiation will help to identify patients at higher risk for grade $\geq$ 2 diarrhea, and could support an a priori dose reduction in order to avoid such toxicity.

In another embodiment of the invention, the method is carried out after the treatment of the patient with sorafenib has been initiated.

In another aspect, the invention also relates to sorafenib for use in a method of treatment of cancer in an individual, wherein said individual does not display the single nucleotide polymorphism (SNP) consisting of *UGT1A9* - 2152 C>T comprising a determination of whether the individual displays the SNP consisting of UGT1A9-2152 C>T.

The invention also relates to the use of sorabenib in the manufacture of a medicament for treating cancer in an individual wherein said individual does not display the single nucleotide polymorphism (SNP) consisting of *UGT1A9* -2152 C>T. , wherein the treatment comprises a determination of whether the individual displays the SNP consisting of UGT1A9-2152 C>T.

The invention further provides a method for treating cancer in an individual in need thereof comprising:

- determining whether said individual displays the single nucleotide polymorphism (SNP) consisting of *UGT1A9* -2152 C>T.

- administering sorafenib to said individual in a suitable amount.

Typically, the suitable amount will be lower for a patient presenting the single nucleotide polymorphism (SNP) consisting of *UGT1A9* -2152 C>T than for an individual presenting the WT allele (-2152 C) (i.e. presenting the nucleotide "C" at position -2152), in order to limit the risk of toxicity of sorafenib.

EXAMPLES:

**METHODS AND MATERIALS**

**1. Patients' selection:**

The investigational review board "Comité de Protection des Personnes d'Ile de France" approved the study protocol (N°AT140); all patients provided written informed consent and approved the sampling and pharmacogenetic analysis in compliance with the ethical principles of the revised Declaration of Helsinki (2008) and with French regulations.

A total of ninety-two consecutive patients with advanced or metastatic solid tumors were treated with single agent sorafenib at the Cochin Teaching Hospital in Paris, France. The study population represents a subgroup of these patients for whom genotyping was accepted and performed. All patients met the following criteria: age $\geq$ 18 years; presence of clinically and/or radiologically assessable disease. Patients with brain metastases were included. As a result, fifty-eight adult patients with advanced solid tumors were enrolled in this monocentric study. Four patients had to be excluded from the analysis: two never began sorafenib treatment (one for severe sepsis, the other died before treatment initiation), one patient did not come to the first follow-up visit and thereafter, and finally one patient for lack of acceptable genotyping success rate due to poor DNA quality. Overall, 54 patients were assessable for toxicity. The clinical and biological baseline characteristics of these patients are summarized in Table 1.

**Table 1:** Clinical and biological characteristics of study group

| Characteristics | N= 54 |
| --- | --- |
| **Demographic data** | |
| Gender, n (%) | |
| Male | 38 (70) |
| Female | 16 (30) |
| Age in years | 64 [58-76] |
| BMI (kg/m$^2$) | 24.0 [22.4-29.0] |
| LBM$^a$ (kg) | 53.1 [50.2-58.6] |
| ECOG performance status, n (%) | |
| 0 | 18 (33) |
| 1 | 22 (41) |
| $\geq$ 2 | 14 (26) |
| **Primary sites, n (%)** | |
| Hepatocellular carcinoma | 20 (37) |
| Melanoma | 13 (24) |
| Differenciated thyroid cancer | 12 (22) |
| Renal cell carcinoma | 6 (11) |
| Other | 3 (6) |

(continued)

| Primary sites, n (%) | |
| --- | --- |
| **Baseline biological data** | |
| AST (UI/L) | 36 [27-68] |
| ALT (UI/L) | 34 [21-51] |
| ALP (UI/L) | 87 [66-186] |
| GGT (UI/L) | 57 [25-182] |
| Bilirubin ($\mu$mol/L) | 10 [7-13] |
| Albumin (g/L) | 39 [34-41] |
| CRP (mg/L) | 7 [3-20] |
| Creatinine Clearance[b] (mL/min) | 69.6 [54.6-82.2] |

ALP, alkaline phosphatase; ALT, Alanine aminotransferase; AST, Aspartate aminotransferase; BMI, body mass index; CRP, C-reactive protein; ECOG, Eastern Cooperative Oncology Group; GGT, Gamma-glutamyl transpeptidase; LBM, Lean Body Mass.

Results are expressed as median [interquartile range].

[a]Lean body mass was estimated by using the Green's formula [29].

[b]Creatinine clearance was estimated by using Modification of Diet in Renal Disease (MDRD) formula [28].

## 2. Treatment plan and toxicity assessment

Patients were started on sorafenib on a twice-daily (bid) schedule (200 mg bid or 400 mg bid depending on their status). During the whole follow-up period, the median sorafenib dose was 800 mg/day (range 200-1200 mg/day). Regarding sorafenib exposure, the median absolute sorafenib AUC was 62.0 [45.4-91.8] mg/L.h and 61.0 [31.6-75.4] mg/L.h on days 15 and 30, respectively. The median absolute AUC in females was significantly greater than that observed in males on day 15 (78.3 [60.4-124.4] vs 52.4 [42.6-81.4] mg/L.h, respectively; p=0.006) and day 30 (82.2 [57.7-108.9] vs 55.2 [31.4-72.5] mg/L.h respectively; p=0.0078). Adverse events were reported and graded according to the National Cancer Institute Common Terminology Criteria (NCI-CTCAE) on days 15 and 30 after treatment initiation. Diarrhea and HFSR were graded according to the NCI-CTCAE 3.0 version [24], while hypertension was retrospectively graded according to the 4.0 version [25]. In case of grade 4 hematological toxicity or grade 3 or 4 non-hematological toxicity, treatment was discontinued until side effects recovered to grade 1, or returned to baseline. Subsequent dose reductions were left at the discretion of the treating physician.

At each follow-up visit, blood samples were drawn into 5-mL lithium heparinized Vacutainer tubes to determine plasma concentrations of sorafenib. After centrifugation at 3,000 rpm for 5 minutes at 4°C, plasma was transferred to propylene tubes and stored at -20°C until assay of plasma sorafenib concentrations.

## 3. Sorafenib pharmacokinetic analysis

Sorafenib concentrations in plasma were determined using a method previously published by our research group [26]. The calibration was linear in the range of 0.5 - 20 mg/L. The accuracy, within-assay and between-assay precision of this method were 96.9-104.0%, 3.4-6.2% and 7.6-9.9%, respectively. A specific Bayesian estimator developed in our institution [27] allowed estimating individual sorafenib area under the plasma concentration-time curve from 0 to 12 hours (AUC) and the average plasma sorafenib concentration (Cav) over 12 hours. This Bayesian estimator is based on a 1-compartment model with saturated absorption, first-order intestinal loss and linear elimination.

## 4. DNA extraction and Genotyping analysis

All enrolled patients were genotyped for 9 polymorphisms in 4 genes potentially involved in the pharmacokinetics of sorafenib. Genotyping was performed in the central laboratory (Hôpital Européen Georges Pompidou, Paris). Genomic DNA was extracted from peripheral blood leukocytes using the QiaAmp DNA mini Kit (Qiagen, Courtaboeuf, France) according to the recommendations of the manufacturer. Following SNPs were identified using Taq Man® Drug Metabolism Genotyping Assays (Applied Biosystems, Courtaboeuf, France): 6986 A>G (rs 776746, C__26201809_30) for CYP3A5, 3435 G>T (rs 1045642, C__7586657_20) and 2677 G>T/A (rs 2032582, C_11711720D_40 and C_11711720C_30) for ABCB1, -275 T>A (rs 6714486, C_27843087_10), -2152 C>T (rs17868320, C_34418857_10), 98 T>C (rs 72551330, C__64627083_10) for *UGT1A9,* 421C>A (rs 2231142, C__15854163_70) and 1143 C>T (rs 2622604, C__9510352_10) for ABCG2. Genotype for ABCG2 34 G>A was determined by sequencing. Its cycling conditions and primers are available on request. Its sequencing was performed on an ABI Prism Genetic Analyzer System 9700 (Applied Biosystems).

Due to DNA quantity or poor DNA quality genotyping, success rates were 98% for CYP3A5*3, UGT1A9 and ABCB1 SNPs and 94% for ABCG2 SNPs.

**5. Statistical analysis**

For descriptive statistics, qualitative variables were expressed in numbers and percentages and quantitative ones in medians [inter-quartile intervals]. Glomerular filtration rate (GFR) and lean body mass (LBM) were estimated using the Modification of the Diet in Renal Disease (MDRD) formula and the Green's formula, respectively [28,29]. To identify the covariates influencing the pharmacokinetics of sorafenib on day 15 after treatment initiation (i.e. once the pharmacokinetic steady-state was reached), the individual absolute AUCs estimated were normalized to a dose of 400 mg sorafenib twice daily. Then, the relationships between the dose-standardized AUC and other variables were tested using two-sample Wilcoxon tests or Kruskal-Wallis tests for qualitative variables with two modalities or more than two modalities, respectively, and by Spearman rank correlation coefficient tests for quantitative ones. The following variables were tested: age, gender, ECOG status, weight, body mass index (BMI), LBM, primary tumor site, polymorphisms of CYP3A5 (rs776746), UGT1A9 (rsl78868320, rs6714486, rs72551330), ABCB1 (rs2231137, rs1045642), ABCG2 (rs2231137, rs2231142, rs2622604), alanine aminotransferase (ALT), aspartate aminotransferase (AST), alkaline phosphatase (ALP), gamma-glutamyl transpeptidase (GGT), bilirubin, albumin, C-Reactive Protein, creatinine and MDRD GFR. A stepwise multiple linear regression was then performed with variables significant with $p < 0.10$ in the first step included in the regression and variables significant with $p < 0.05$ kept in the final model. For the analysis of sorafenib-induced toxicities in the first month of treatment, we used dichotomous end points expressed as any toxicity $\geq$ grade 3 (yes or no), or grade $\geq$ 2 HFSR (yes or no), or grade $\geq$ 2 diarrhea (yes or no), or grade $\geq$ 2 hypertension (yes or no). The cumulated AUC between day 0 and day 30 was calculated as the sum of two areas, (0-15 days) and (15-30 days) which were estimated using the trapezoidal rule. The area was multiplied by 24 to express the result in mg/L.h.

The cumulated AUC (AUCcum) on day 30 was calculated according to the following formula:

$$AUCcum = [(\frac{(Cav_0 + Cav_{15})}{2} \times 24) \times (D15 - D0)] + [(\frac{(Cav_{15} + Cav_{30})}{2} \times 24) \times (D30 - D15)]$$

where Cav and D are average plasma sorafenib concentrations over 12 hours and days, respectively. On day 0, Cav0 was fixed at 0 mg/L.

The univariate analysis of toxicity risk factors was carried out using two sample Wilcoxon tests for quantitative variables and Chi square or Fisher exact tests for qualitative ones. Fisher exact tests were performed as soon as at least one expected value was lower than 5. The following variables were tested: age, gender, ECOG status, weight, BMI, LBM, primary tumor site, polymorphisms of CYP3A5 (rs776746), UGT1A9 (rs178868320, rs6714486, rs72551330), ABCB1 (rs2231137, rs1045642), ABCG2 (rs2231137, rs2231142, rs2622604), ALT, AST, ALP, GGT, bilirubin, albumin, CRP, creatinine, MDRD GFR, absolute sorafenib AUC and AUCcum. The multivariate analysis was performed by a stepwise logistic regression. Variables with p-values $< 0.10$ in the univariate analysis were included in the regression and variables with a Wald test p-value $< 0.05$ were kept in the final model. All the tests were two-sided, and they were considered significant when p-values were $< 0.05$. The receiver operating characteristic (ROC) curve was constructed to examine the value of an AUCcum threshold predicting a toxicity of grade $\geq$ 3 in the first month of treatment. The diagnostic accuracy values of sensitivity and specificity were determined, as well as the positive predictive value (PPV) and the negative predictive value (NPV). Computations were performed using the SAS V9 statistical package (SAS institute, Cary, NC).

**RESULTS**

**1. Genotype distribution**

Table 2 presents the genotype distribution of the different polymorphisms. Variant allele frequencies estimated on the entire series of 54 patients were in accordance with those observed in the Caucasian population. Genotypes were distributed according to the Hardy Weinberg equilibrium except for *CYP3A5* and *UGT1A9* (98 T>C). For *CYP3A5,* a greater than expected number of the variant *CYP3A5\*1\*1* was observed. However this deviation was not observed when considering only our Caucasian patients, who represented 87% of our population. None of the patients carried the variants for *UGT1A9* 98 T>C. For - 2152 C > T, around 7.5% of the 54 patients carried this SNP, which conforms with the former observations [42].

**Table 2:** Genotype distribution and minor allele frequency among the 54 included patients.

| Gene | Identification Number | SNPs | Genotype | | | |
|---|---|---|---|---|---|---|
| | | | wt/wt n (%) | wt/m n (%) | m/m n (%) | Minor Allele Frequency |
| *CYP3A5* | | | | | | |
| | rs776746 | 6986 A > G | 4 (7) | 11 (20) | 39 (73) | 0.185[a] |
| *UGT1A9* | | | | | | |
| | rs17868320 | − 2152 C > T | 50 (92) | 4 (8) | 0 (0) | 0.075 |
| | rs6714486 | −275 T > A | 48 (90) | 5 (10) | 0 (0) | 0.096 |
| | rs72551330 | 98 T > C | 54 (100) | 0 (0) | 0 (0) | 0 |
| *ABCB1* | | | | | | |
| | rs2032582 | 3435C > T | 17 (32) | 25 (46) | 12 (22) | 0.452 |
| | rs1045642 | 2677 G > T/A | 22 (41) | 22 (41) | 10 (18) | 0.415 |
| *ABCG2* | | | | | | |
| | rs2231137 | 34 G > A | 41 (79) | 10 (19) | 1 (2) | 0.117 |
| | rs2231142 | 421 C>A | 42 (81) | 8 (15) | 2 (4) | 0.117 |
| | rs2622604 | 1143 C > T | 32 (61) | 16 (31) | 4 (8) | 0.235 |

m, mutant allele, SNP, Single-Nucleotide Polymorphism; wt, wild-type allele.
[a] the less common allele for CYP3A5 was the A-allele (wild type allele) in our predominantly Caucasian population

### 2. Factors influencing sorafenib exposure

There was wide interindividual variability in dose-normalized exposure to sorafenib on day 15 (n=51; CV=58.0%), and the median dose-normalized AUC was 78.0 [51.0-114.8] mg/L.h. The median dose-normalized AUC in females was 2.1-fold greater than that observed in males (136.0 vs 64.8 mg/L.h, respectively, p=0.0008) (Table 3). CYP3A5\*1\*1 carriers presented a greater exposure than CYP3A5\*3\*3 carriers (136.0 mg vs 67.4 mg/L.h, respectively), related to the predominance of females in CYP3A5\*1\*1 carriers (80%) compared to CYP3A5\*3\*3 (29%) carriers. Patients with the ABCG2 1143 TT genotype exhibited a greater exposure compared to those with the ABCG2 1143 CC genotype (131.8 vs 82.4 mg/L.h, respectively). The multiple linear regression showed that gender (p=0.0008) was the single parameter independently associated with the dose-normalized exposure to sorafenib on day 15 after treatment initiation.

**Table 3:** Genetic and non-genetic factors influencing exposure to sorafenib on day 15 after treatment initiation

| Variable | Sorafenib AUC [a] (mg/L.h) | Spearman's coefficient (rho) | P value | Linear regression P value |
|---|---|---|---|---|
| | | Univariate analysis | | |
| Gender | | | **0.011** | **0.0008** |
| Female | 136.0 [60.4-182.5] | | | |
| Male | 64.8 [45.4-94.2] | | | |
| *CYP3A5* 6986 A>G | | | 0.063 | NS |
| GG (*3/*3) | 67.4 [51.0-124.0] | | | |
| AG (*1/*3) | 75.0 [43.1-90.4] | | | |
| AA (*1/*1) | 136.0 [117.8-168.81 | | | |
| *ABCG2* 1143 C>T | | | 0.093 | NS |
| CC | 82.4 [52.2-139.9] | | | |
| CT | 64.8 [41.3-103.1] | | | |
| TT | 131.8 [96.0-170.1] | | | |
| LBM[b] (kg) | | -0.28 | 0.053 | NS |
| Creatinine ($\mu$mol/L) | | -0.26 | 0.076 | NS |

AUC, area under the curve; LBM, Lean Body Mass Results are expressed median [interquartile range]

[a] AUC was dose-normalized per 400 mg of sorafenib

[b] Lean body mass was estimated by using the Green's formula [29] Data were available for 51 patients

## 3. Toxicity

In the present cohort of patients, toxicities occurring during the first month of sorafenib therapy were used as outcome measures for different reasons. Firstly, the screening of pharmacogenetic variants within the routine pre-therapeutic evaluation is likely to be more relevant to predict early severe toxicities. Secondly, patients from a "real life" population are frailer compared to those selected in clinical trials, and therefore more vulnerable to developing severe toxicities. Finally, signs of clinical deterioration due to the disease progression over time could be misinterpreted and could interfere with the drug-induced toxicity outcome.

The most common grade 2-3 events were HFSR in 22 (41%) of the 54 patients, hypertension in 20 (38%) and diarrhea in 5 (9%) (Table 4). Twenty-two patients (41%) experienced grade 3 or 4 toxicity on days 15 or 30: HFSR (n=15, 68%), followed by hypertension (n=6, 27%), and diarrhea (n=1, 5%). One patient exhibited grade 3 hypertension on day 15, followed by a grade 3 HFSR on day 30. Nineteen patients (35%) required dose reductions (n=9) or interruption of treatment (n=10) because of toxic effects over the study period. Finally, there was no toxic death in relation with sorafenib.

**Table 4:** Distribution of increased toxicity grades occurring during the first month of therapy.

| Toxicity by grade | Occurrence during first month of treatment n (%) |
|---|---|
| **Hand-foot skin reaction** | |
| No | 17 (31) |
| Yes | 37 (69) |
| < 2 | 15 |
| $\geq$ 2 | 22 |
| **Hypertension** | |
| No | 28 (52) |
| Yes | 25 (46) |
| < 2 | 5 |
| $\geq$ 2 | 20 |
| NA | 1 |

(continued)

| Diarrhea | |
|---|---|
| No | 37 (69) |
| Yes | 17 (31) |
| < 2 | 12 |
| ≥ 2 | 5 |

## 4. Risk factors for any grade ≥ 3 toxicity and grade ≥ 2 HFSR, diarrhea and hypertension

Table 5 shows that increased AUCcum was the sole parameter independently associated with any grade ≥ 3 toxicity (OR: 1.07; 95% CI, 1.01-1.12; p=0.037). Regarding specific toxicities, female sex was identified as an independent risk factor for developing grade ≥ 2 HFSR (OR: 5.26; 95% CI, 1.33-20.0; p=0.018), and the presence of the mutant T allele in *UGT1A9* - 2152 C>T for grade ≥ 2 diarrhea (OR: 14.33; 95% CI, 1.46-140.50; p=0.015) (Table 5). In contrast, no independent risk factor was identified for the development of grade ≥ 2 hypertension.

**Table 5:** Risk factors associated with sorafenib-induced toxicity, defined as any toxicity ≥ grade 3 or hand foot skin reaction, hypertension and diarrhea grade ≥ 2

| | Univariate analysis | | | Multivariate analysis | |
|---|---|---|---|---|---|
| Variable | No | Yes | *p*- value | Odds ratio (95% CI) | *p*- value |
| **Any toxicity ≥ grade 3** | | | | | |
| Gender | | | 0.024 | | NS |
| Female, n (%) | 5 (33.3) | 10 (66.7) | | | |
| Male, n (%) | 25 (67.6) | 12 (32.4) | | | |
| *CYP 3A5* 6986 A>G, n(%) | | | 0.05 | | NS |
| GG (*3/*3) | 24 (63) | 14 (37) | | | |
| AG (*1/*3) | 6 (67) | 3 (33) | | | |
| AA (*1/*1) | 0 (0) | 4 (100) | | | |
| Cumulated sorafenib AUC (mg/L.h) | 2,250 [1,845-2,858] | 3,499 [2,070-4,019] | 0.034 | **1.07 (1.01-1.12)** | **0.019** |
| **Hand Foot Skin Reaction ≥ grade 2** | | | | | |
| Gender | | | 0.024 | **5.26 (1.33-20.0)** | **0.018** |
| Female, n (%) | 5 (33.3) | 10 (66.7) | | | |
| Male, n (%) | 25 (52.0) | 12 (48.0) | | | |
| ECOG PS | 1 [1-2] | 1 [0-1] | 0.043 | | NS |
| Cumulated sorafenib AUC (mg/L.h) | 2,250 [1,789-2,858] | 3,308 [2,070-4,365] | 0.042 | | NS |
| **Diarrhea ≥ grade 2** | | | | | |
| *UGT1A9* -275 T>A | | | 0.043 | | NT* |
| wt/wt, n (%) | 43 (93) | 3 (7) | | | |
| wt/m, n (%) | 2 (50) | 2 (50) | | | |
| m/m, n (%) | 0 (0) | 0 (0) | | | |
| *UGT1A9* -2152 C>T | | | 0.045 | **14.33 (1.46-140.50)** | **0.015** |
| wt/wt, n (%) | 42 (93) | 3 (7) | | | |

(continued)

| Diarrhea ≥ grade 2 | | |
|---|---|---|
| wt/m, n (%) | 2 (50) | 2 (50) |
| m/m, n (%) | 0 (0) | 0 (0) |

| Hypertension ≥ grade 2 | | | | |
|---|---|---|---|---|
| Albumin (g/L) | 37 [34-40] | 41 [37-42] | 0.06 | NS |

AUC, area under the curve; BMI, body mass index; ECOG PS, Eastern Cooperative Oncology Group Performance Status; m, mutant allele; NS, not significant; NT, not tested; Wt, wild-type allele. Quantitative results are expressed as median [interquartile range] *Heterozygotous patients (wt/m) for *UGT1A9*-2152 C>T and *UGT1A9*-275 T>A were the same; therefore the polymorphism for *UGT1A9*-275 T>A was not tested in the multivariate analysis

### 5. Determination of a threshold for sorafenib AUCcum

Using the ROC curve, the AUCcum threshold predicting a toxicity of grader 3 was 3,161 mg/L.h with the area under the curve at 69% (CI 95%, 61-78%; p=0.018) in our population. Considering the threshold value of 3,161 mg/L.h, PPV and NPV were 75.8% and 63.3%, respectively.

### 6. Conclusion

The present results confirm the large interindividual variability in sorafenib exposure [8,27,33].

For the first time, we clearly shows by these experiments that the SNP *UGT1A9* -2152 C>T is associated with the risk of grade ≥ 2 diarrhea upon exposure to sorafenib.

Moreover, we provide the first evidence that an increased cumulated sorafenib exposure is associated with an increased risk of grade ≥ 3 toxicity in the first month of treatment. Besides, a cumulated sorafenib AUC of 3,161 mg/L.h seems to be a significant threshold for developing any grade ≥ 3 toxicity. However, further studies with larger cohorts are required to determine optimal threshold value before its use in daily clinical routine to prevent the occurrence of grade ≥ 3 toxicities and thereby to avoid a transient interruption or a definitive discontinuation of sorafenib therapy.

In disagreement with two population pharmacokinetic studies [8, 27], we observed an influence of gender on sorafenib dose-normalized exposure on day 15 after treatment initiation since females were overexposed compared to males.

Hand-foot skin reaction was the most prevalent toxicity in the present study, which is in consistence with the shorter delay of occurrence of this toxicity compared to diarrhea and hypertension. In the present cohort, the risk for developing grade ≥2 HFSR in the first month of sorafenib therapy was approximately 5-fold greater in females compared to males (OR 5.26). Finally, the present study highlights for the first time that greater sorafenib exposure in females compared to males over the first month of therapy may be a determining factor of sorafenib-induced HFSR in females.

In conclusion, the SNP *UGT1A9* -2152 C>T is a useful index for determining the risk of toxicity, and in particular, the risk of grade ≥ 2 diarrhea upon exposure to sorafenib. Additionally, the female gender is also another factor for aggravating sorafenib toxicity, notably by inducing HFSR.

### References

1. Wilhelm SM, Adnane L, Newell P, Villanueva A, Llovet JM, et al. (2008) Preclinical overview of sorafenib, a multikinase inhibitor that targets both Raf and VEGF and PDGF receptor tyrosine kinase signaling. Mol Cancer Ther 7: 3129-3140.

2. Iyer R, Fetterly G, Lugade A, Thanavala Y (2011) Sorafenib: a clinical and pharmacologic review. Expert Opin Pharmacother 11: 1943-1955.

3. Blanchet B, Billemont B, Barete S, Garrigue H, Cabanes L, et al. (2010) Toxicity of sorafenib: clinical and molecular aspects. Expert Opin Drug Saf 9: 275-287.

4. Porta C, Paglino C, Imarisio I, Bonomi L (2007) Uncovering Pandora's vase: the growing problem of new toxicities from novel anticancer agents. The case of sorafenib and sunitinib. Clin Exp Med 7: 127-134.

5. van Erp NP, Gelderblom H, Guchelaar HJ (2009) Clinical pharmacokinetics of tyrosine kinase inhibitors. Cancer Treat Rev 35: 692-706.

6. European Medicines A Sorafenib (Nexavar): summary of product characteristics [online]. Available from URL: http://www.ema.europa.eu/humandocs/PDFs/EPAR/nexavar/H-690-en6.pdf.

7. Tod M, Mir O, Bancelin N, Coriat R, Thomas-Schoemann A, et al. (2011) Functional and clinical evidence of the influence of sorafenib binding to albumin on sorafenib disposition in adult cancer patients. Pharm Res 28: 3199-3207.

8. Jain L, Woo S, Gardner ER, Dahut WL, Kohn EC, et al. (2011) Population pharmacokinetic analysis of sorafenib in patients with solid tumours. Br J Clin Pharmacol 72: 294-305.

9. Strumberg D, Richly H, Hilger RA, Schleucher N, Korfee S, et al. (2005) Phase I clinical and pharmacokinetic study of the Novel Raf kinase and vascular endothelial growth factor receptor inhibitor BAY 43-9006 in patients with advanced refractory solid tumors. J Clin Oncol 23: 965-972.

10. Kuypers DR, Naesens M, Vermeire S, Vanrenterghem Y (2005) The impact of uridine diphosphate-glucuronosyltransferase 1A9 (UGT1A9) gene promoter region single-nucleotide polymorphisms T-275A and C-2152T on early mycophenolic acid dose-interval exposure in de novo renal allograft recipients. Clin Pharmacol Ther 78: 351-361.

11. Villeneuve L, Girard H, Fortier LC, Gagne JF, Guillemette C (2003) Novel functional polymorphisms in the UGT1A7 and UGT1A9 glucuronidating enzymes in Caucasian and African-American subjects and their impact on the metabolism of 7-ethyl-10-hydroxycamptothecin and flavopiridol anticancer drugs. J Pharmacol Exp Ther 307: 117-128.

12. Agarwal S, Sane R, Ohlfest JR, Elmquist WF (2011) The role of the breast cancer resistance protein (ABCG2) in the distribution of sorafenib to the brain. J Pharmacol Exp Ther 336: 223-233.

13. Lagas JS, van Waterschoot RA, Sparidans RW, Wagenaar E, Beijnen JH, et al. (2010) Breast cancer resistance protein and P-glycoprotein limit sorafenib brain accumulation. Mol Cancer Ther 9: 319-326.

14. van Erp NP, Eechoute K, van der Veldt AA, Haanen JB, Reyners AK, et al. (2009) Pharmacogenetic pathway analysis for determination of sunitinib-induced toxicity. J Clin Oncol 27: 4406-4412.

15. Rudin CM, Liu W, Desai A, Karrison T, Jiang X, et al. (2008) Pharmacogenomic and pharmacokinetic determinants of erlotinib toxicity. J Clin Oncol 26: 1119-1127.

16. Li J, Cusatis G, Brahmer J, Sparreboom A, Robey RW, et al. (2007) Association of variant ABCG2 and the pharmacokinetics of epidermal growth factor receptor tyrosine kinase inhibitors in cancer patients. Cancer Biol Ther 6: 432-438.

17. Lemos C, Giovannetti E, Zucali PA, Assaraf YG, Scheffer GL, et al. (2011) Impact of ABCG2 polymorphisms on the clinical outcome and toxicity of gefitinib in non-small-cell lung cancer patients. Pharmacogenomics 12: 159-170.

18. Cusatis G, Gregorc V, Li J, Spreafico A, Ingersoll RG, et al. (2006) Pharmacogenetics of ABCG2 and adverse reactions to gefitinib. J Natl Cancer Inst 98: 1739-1742.

19. Garcia-Donas J, Esteban E, Leandro-Garcia LJ, Castellano DE, Del Alba AG, et al. (2011) Single nucleotide polymorphism associations with response and toxic effects in patients with advanced renal-cell carcinoma treated with first-line sunitinib: a multicentre, observational, prospective study. Lancet Oncol 12: 1143-1150.

20. Lu JF, Eppler SM, Wolf J, Hamilton M, Rakhit A, et al. (2006) Clinical pharmacokinetics of erlotinib in patients with solid tumors and exposure-safety relationship in patients with non-small cell lung cancer. Clin Pharmacol Ther 80: 136-145.

21. Thomas F, Rochaix P, White-Koning M, Hennebelle I, Sarini J, et al. (2009) Population pharmacokinetics of erlotinib and its pharmacokinetic/pharmacodynamic relationships in head and neck squamous cell carcinoma. Eur J Cancer 45: 2316-2323.

22. White-Koning M, Civade E, Geoerger B, Thomas F, Le Deley MC, et al. (2011) Population analysis of erlotinib in adults and children reveals pharmacokinetic characteristics as the main factor explaining tolerance particularities in children. Clin Cancer Res 17: 4862-4871.

23. Houk BE, Bello CL, Poland B, Rosen LS, Demetri GD, et al. (2011) Relationship between exposure to sunitinib and efficacy and tolerability endpoints in patients with cancer: results of a pharmacokinetic/pharmacodynamic meta-analysis. Cancer Chemother Pharmacol 66: 357-371.

24. National Cancer Institute. Common Terminology Criteria for Adverse Events VAfhccg, Publish Date: August 9, 2006. 2006.

25. National Cancer Institute. Common Terminology Criteria for Adverse Events VAfhccg, Publish Date: May 28, 2009. 2009.

26. Blanchet B, Billemont B, Cramard J, Benichou AS, Chhun S, et al. (2009) Validation of an HPLC-UV method for sorafenib determination in human plasma and application to cancer patients in routine clinical practice. J Pharm Biomed Anal 49: 1109-1114.

27. Hornecker M, Blanchet B, Billemont B, Sassi H, Ropert S, et al. (2011) Saturable absorption of sorafenib in patients with solid tumors: a population model. Invest New Drugs: Oct 18 [Epub ahead of print].

28. Levey AS, Coresh J, Greene T, Marsh J, Stevens LA, et al. (2007) Expressing the Modification of Diet in Renal Disease Study equation for estimating glomerular filtration rate with standardized serum creatinine values. Clin Chem 53: 766-772.

29. Janmahasatian S, Duffull SB, Ash S, Ward LC, Byrne NM, et al. (2005) Quantification of lean bodyweight. Clin Pharmacokinet 44: 1051-1065.

30. Morimoto M, Numata K, Kondo M, Hidaka H, Takada J, et al. (2011) Higher discontinuation and lower survival rates are likely in elderly Japanese patients with advanced hepatocellular carcinoma receiving sorafenib. Hepatol Res 41: 296-302.

31. Azad NS, Aragon-Ching JB, Dahut WL, Gutierrez M, Figg WD, et al. (2009) Hand-foot skin reaction increases with cumulative sorafenib dose and with combination anti-vascular endothelial growth factor therapy. Clin Cancer Res 15: 1411-1416.

32. Dranitsaris G, Vincent MD, Yu J, Huang L, Fang F, et al. (2012) Development and validation of a prediction index for hand-foot skin reaction in cancer patients receiving sorafenib. Annals of Oncology: January 6 [Epub ahead of print].

33. Strumberg D, Clark JW, Awada A, Moore MJ, Richly H, et al. (2007) Safety, pharmacokinetics, and preliminary antitumor activity of sorafenib: a review of four phase I trials in patients with advanced refractory solid tumors. Oncologist 12: 426-437.

34. Prado CM, Lima IS, Baracos VE, Bies RR, McCargar LJ, et al. (2011) An exploratory study of body composition as a determinant of epirubicin pharmacokinetics and toxicity. Cancer Chemother Pharmacol 67: 93-101.

35. Mathijssen RH, Sparreboom A (2009) Influence of lean body weight on anticancer drug clearance. Clin Pharmacol Ther 85: 23; author reply 24.

36. Mourtzakis M, Prado CM, Lieffers JR, Reiman T, McCargar LJ, et al. (2008) A practical and precise approach to quantification of body composition in cancer patients using computed tomography images acquired during routine care. Appl Physiol Nutr Metab 33: 997-1006.

37. Gnoth MJ, Sandmann S, Engel K, Radtke M (2010) In vitro to in vivo comparison of the substrate characteristics of sorafenib tosylate toward P-glycoprotein. Drug Metab Dispos 38: 1341-1346.

38. Delbaldo C, Chatelut E, Re M, Deroussent A, Seronie-Vivien S, et al. (2006) Pharmacokinetic-pharmacodynamic relationships of imatinib and its main metabolite in patients with advanced gastrointestinal stromal tumors. Clin Cancer Res 12: 6073-6078.

39. Treinen-Moslen M, Kanz MF (2006) Intestinal tract injury by drugs: Importance of metabolite delivery by yellow bile road. Pharmacol Ther 112: 649-667.

40. Gomo C, Coriat R, Faivre L, Mir O, Ropert S, et al. (2011) Pharmacokinetic interaction involving sorafenib and the calcium-channel blocker felodipine in a patient with hepatocellular carcinoma. Invest New Drugs 29: 1511-1514.

41. http://www.pharmacogenomics.pha.ulaval.ca/files/content/ sites/pharmacogenomics/files/Nomenclature/UGT1A/UGT1A9.htm

42. Sanchez-Fructuoso et al. (2009) The prevalence of Uridine Diphosphate-Glucuronosyltransferase 1A9 (UGT1A9) Gene Promoter Region Single-Nucleotide Polymorphisms T-275A and C-2152T and its influence on Mycophenolic Acid Pharmacokinetics in Stable Renal Transplant Patients, Transplantation Proceedings, 41 2313-2316

43. Shim et al. (2011) Genetic predisposition of hand-foot skin reaction following sorafenib therapy in Korean patients with hepatocellular carcinoma, J Hepatol 54(suppl. 1):S264.

## Claims

1. An in vitro method for determining the risk of toxicity of sorafenib in an individual upon sorafenib intake, comprising analyzing the nucleotide sequence of the *UGT1A9* gene for the presence of the single nucleotide polymorphism (SNP) consisting of *UGT1A9* -2152 C>T, in a biological sample obtained from said individual, wherein said toxicity comprises suffering from diarrhea.

2. The method according to claim 1, wherein the presence of *UGT1A9* -2152 C>T is indicative of a greater risk of toxicity upon sorafenib intake.

3. The method according to any of the above claims, wherein the individual is of female gender.

4. The method according to any of the above claims, wherein said *UGT1A9* gene further displays at least one SNP selected from the group consisting of -440 T>C, -331 C>T, -275 T>A, - 665 C>T, -5366 G>T, -4549 T>C, I219 A>T, I313 C>A and I399 C>T.

5. The method according to any of the above claims, wherein said *UGT1A9* gene comprises one or several alleles selected among *UGT1A9*\*1L and *UGT1A9*\*1N.

6. The method according to any of the above claims, wherein the severity grade of said diarrhea is at least 2 according to the NCI-CTCAE 3.0 grading version.

7. The method according to any of the above claims, wherein the sample is selected from the group consisting of blood, semen, saliva, tears, urine, fecal material, sweat, buccal cells, skin, hair and other tissue containing nucleic acid of the individual.

8. The method according to any of the above claims, wherein the sample contains peripheral blood leukocytes.

9. The method according to any of the above claims, wherein said individual suffers from cancer.

10. The method according to claim 9, wherein said individual suffers from renal, liver, kidney, thyroid, lung or brain cancer.

11. The method according to any of the claims 9-10, wherein said individual suffers from an advanced or metastatic solid tumor.

**12.** The method according to any of the claims 9-11, wherein said individual suffers from advanced renal cell carcinoma or unresectable hepatocellular carcinoma.

**13.** Sorafenib for use in a method of treatment of cancer in an individual, wherein said method comprises:

- determining whether said individual displays the single nucleotide polymorphism (SNP) consisting of *UGT1A9* - 2152 C>T; and
- if said individual does not display the single nucleotide polymorphism (SNP) consisting of *UGT1A9* -2152 C>T, administering sorafenib to said individual.

**Patentansprüche**

**1.** In vitro-Verfahren zur Bestimmung des Toxizitätsrisikos von Sorafenib in einem Individuum nach Sorafenib-Aufnahme, umfassend die Untersuchung der Nucleotidsequenz des *UGT1A9*-Gens auf das Vorliegen des Einzelnucleotid-Polymorphismus (SNP), der aus *UGT1A9* -2152 C>T besteht, in einer biologischen Probe, die von dem Individuum erhalten wurde, wobei die Toxizität das Erkranken an Diarrhöe umfasst.

**2.** Verfahren nach Anspruch 1, wobei das Vorliegen von *UGT1A9* -2152 C>T auf ein erhöhtes Toxizitätsrisiko nach Sorafenib-Aufnahme schließen lässt.

**3.** Verfahren nach einem der vorstehenden Ansprüche, wobei das Individuum weiblichen Geschlechts ist.

**4.** Verfahren nach einem der vorstehenden Ansprüche, wobei das *UGT1A9*-Gen zudem mindestens einen SNP aufweist, der ausgewählt ist aus der Gruppe bestehend aus -440 T>C, -331 C>T, -275 T>A, -665 C>T, -5366 G>T, -4549 T>C, I219 A>T, I313 C>A und I399 C>T.

**5.** Verfahren nach einem der vorstehenden Ansprüche, wobei das *UGT1A9*-Gen zudem ein oder mehrere Allele umfasst, die ausgewählt sind aus *UGT1A9*\*1L und *UGT1A9*\*1 N.

**6.** Verfahren nach einem der vorstehenden Ansprüche, wobei der Schweregrad der Diarrhöe mindestens 2 gemäß der NCI-CTCAE 3.0 Bewertungsversion ist.

**7.** Verfahren nach einem der vorstehenden Ansprüche, wobei die Probe ausgewählt ist aus der Gruppe bestehend aus Blut, Samen, Speichel, Tränen, Urin, fäkalem Material, Schweiß, bukkalen Zellen, Haut, Haar und anderem Gewebe, das Nucleinsäure des Individuums enthält.

**8.** Verfahren nach einem der vorstehenden Ansprüche, wobei die Probe Leukozyten des peripheren Blutes enthält.

**9.** Verfahren nach einem der vorstehenden Ansprüche, wobei das Individuum an Krebs leidet.

**10.** Verfahren nach Anspruch 9, wobei das Individuum an renalem Krebs, Leber-, Nieren-, Schilddrüsen-, Lungen- oder Gehirnkrebs leidet.

**11.** Verfahren nach einem der Ansprüche 9 bis 10, wobei das Individuum an einem fortgeschrittenen oder metastasierenden soliden Tumor leidet.

**12.** Verfahren nach einem der Ansprüche 9 bis 11, wobei das Individuum an fortgeschrittenem Nierenzellkarzinom oder inoperablem Leberzellkarzinom leidet.

**13.** Sorafenib zur Verwendung in einem Verfahren zur Behandlung von Krebs in einem Individuum, wobei das Verfahren umfasst:

- Bestimmen, ob das Individuum den Einzelnucleotid-Polymorphismus (SNP) bestehend aus *UGT1A9* -2152 C>T aufweist; und
- wenn das Individuum den Einzelnucleotid-Polymorphismus (SNP) bestehend aus *UGT1A9* -2152 C>T nicht aufweist, Verabreichen von Sorafenib an das Individuum.

**Revendications**

1. Procédé *in vitro* pour déterminer le risque de toxicité du sorafénib chez un individu lors d'une consommation de sorafénib, comprenant l'analyse de la séquence nucléotidique du gène *UGT1A9* pour la présence d'un polymorphisme simple nucléotide (SNP) consistant en *UGT1A9* - 2152 C>T, dans un échantillon biologique obtenu chez l'individu, dans lequel la toxicité comprend le fait d'être atteint de diarrhée.

2. Procédé selon la revendication 1, dans lequel la présence de *UGT1A9* - 2152 C>T est indicatif d'un risque accru de toxicité lors d'une consommation de sorafénib.

3. Procédé selon l'une quelconque des revendications précédentes, dans lequel l'individu est de sexe féminin.

4. Procédé selon l'une quelconque des revendications précédentes, dans lequel le gène *UGT1A9* présente en outre au moins un SNP sélectionné dans le groupe constitué par -440 T>C, -331 C>T, -275 T>A, -665 C>T, -5366 G>T, -4549 T>C, I219 A>T, I313 C>A et 1399 C>T.

5. Procédé selon l'une quelconque des revendications précédentes, dans lequel le gène *UGT1A9* comprend un plusieurs allèles choisis parmi *UGT1A9*\*1L *et UGT1A9*\*1N.

6. Procédé selon l'une quelconque des revendications précédentes, dans lequel le grade de sévérité de ladite diarrhée est d'au moins 2 selon l'échelle NCI-CTCAE version 3.0.

7. Procédé selon l'une quelconque des revendications précédentes, dans lequel l'échantillon est sélectionné dans le groupe constitué par le sang, le sperme, la salive, les larmes, l'urine, la matière fécale, la sueur, les cellules buccales, la peau, les cheveux et les autres tissus contenant de l'acide nucléique de l'individu.

8. Procédé selon l'une quelconque des revendications précédentes, dans lequel l'échantillon contient des leucocytes de sang circulant.

9. Procédé selon l'une quelconque des revendications précédentes, dans lequel l'individu est atteint de cancer.

10. Procédé selon la revendication 9, dans lequel l'individu est atteint de cancer rénal, du foie, du rein, de la thyroïde, des poumons ou du cerveau.

11. Procédé selon l'une quelconque des revendications 9-10, dans lequel l'individu est atteint d'une tumeur solide avancée ou métastatique.

12. Procédé selon l'une quelconque des revendications 9-11, dans lequel l'individu est atteint de carcinome rénal avancé ou de carcinome hépatocellulaire inopérable.

13. Sorafénib pour une utilisation dans une méthode de traitement du cancer chez un individu, ladite méthode comprenant :

    - déterminer si ledit individu possède le polymorphisme simple nucléotide (SNP) consistant en *UGT1A9* -2152 C>T ; and
    - si ledit individu ne possède pas le polymorphisme simple nucléotide (SNP) consistant en *UGT1A9* -2152 C>T, administrer du sorafénib audit individu.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WILHELM SM ; ADNANE L ; NEWELL P ; VIL-LANUEVA A ; LLOVET JM et al.** Preclinical overview of sorafenib, a multikinase inhibitor that targets both Raf and VEGF and PDGF receptor tyrosine kinase signaling. *Mol Cancer Ther,* 2008, vol. 7, 3129-3140
- **IYER R ; FETTERLY G ; LUGADE A ; THANAVALA Y.** Sorafenib: a clinical and pharmacologic review. *Expert Opin Pharmacother,* 2011, vol. 11, 1943-1955
- **BLANCHET B ; BILLEMONT B ; BARETE S ; GAR-RIGUE H ; CABANES L et al.** Toxicity of sorafenib: clinical and molecular aspects. *Expert Opin Drug Saf,* 2010, vol. 9, 275-287
- **PORTA C ; PAGLINO C ; IMARISIO I ; BONOMI L.** Uncovering Pandora's vase: the growing problem of new toxicities from novel anticancer agents. The case of sorafenib and sunitinib. *Clin Exp Med,* 2007, vol. 7, 127-134
- **VAN ERP NP ; GELDERBLOM H ; GUCHELAAR HJ.** Clinical pharmacokinetics of tyrosine kinase inhibitors. *Cancer Treat,* 2009, vol. 35, 692-706
- *European Medicines A Sorafenib (Nexavar): summary of product characteristics, http://www.ema.europa.eu/humandocs/PDFs/EPAR/nexavar/H-690-en6.pdf*
- **TOD M ; MIR O ; BANCELIN N ; CORIAT R ; THO-MAS-SCHOEMANN A et al.** Functional and clinical evidence of the influence of sorafenib binding to albumin on sorafenib disposition in adult cancer patients. *Pharm Res,* 2011, vol. 28, 3199-3207
- **JAIN L ; WOO S ; GARDNER ER ; DAHUT WL ; KOHN EC et al.** Population pharmacokinetic analysis of sorafenib in patients with solid tumours. *Br J Clin Pharmacol,* 2011, vol. 72, 294-305
- **STRUMBERG D ; RICHLY H ; HILGER RA ; SCH-LEUCHER N ; KORFEE S et al.** Phase I clinical and pharmacokinetic study of the Novel Raf kinase and vascular endothelial growth factor receptor inhibitor BAY 43-9006 in patients with advanced refractory solid tumors. *J Clin Oncol,* 2005, vol. 23, 965-972

- **KUYPERS DR ; NAESENS M ; VERMEIRE S ; VAN-RENTERGHEM Y.** The impact of uridine diphosphate-glucuronosyltransferase 1A9 (UGT1A9) gene promoter region single-nucleotide polymorphisms T-275A and C-2152T on early mycophenolic acid dose-interval exposure in de novo renal allograft recipients. *Clin Pharmacol Ther,* 2005, vol. 78, 351-361
- **VILLENEUVE L ; GIRARD H ; FORTIER LC ; GAGNE JF ; GUILLEMETTE C.** Novel functional polymorphisms in the UGT1A7 and UGT1A9 glucuronidating enzymes in Caucasian and African-American subjects and their impact on the metabolism of 7-ethyl-10-hydroxycamptothecin and flavopiridol anticancer drugs. *J Pharmacol Exp Ther,* 2003, vol. 307, 117-128
- **AGARWAL S ; SANE R ; OHLFEST JR ; ELMQUIST WF.** The role of the breast cancer resistance protein (ABCG2) in the distribution of sorafenib to the brain. *J Pharmacol Exp Ther,* 2011, vol. 336, 223-233
- **LAGAS JS ; VAN WATERSCHOOT RA ; SPARI-DANS RW ; WAGENAAR E ; BEIJNEN JH et al.** Breast cancer resistance protein and P-glycoprotein limit sorafenib brain accumulation. *Mol Cancer Ther,* 2010, vol. 9, 319-326
- **VAN ERP NP ; EECHOUTE K ; VAN DER VELDT AA ; HAANEN JB ; REYNERS AK et al.** Pharmacogenetic pathway analysis for determination of sunitinib-induced toxicity. *J Clin Oncol,* 2009, vol. 27, 4406-4412
- **RUDIN CM ; LIU W ; DESAI A ; KARRISON T ; JIANG X et al.** Pharmacogenomic and pharmacokinetic determinants of erlotinib toxicity. *J Clin Oncol,* 2008, vol. 26, 1119-1127
- **LI J ; CUSATIS G ; BRAHMER J ; SPARREBOOM A ; ROBEY RW et al.** Association of variant ABCG2 and the pharmacokinetics of epidermal growth factor receptor tyrosine kinase inhibitors in cancer patients. *Cancer Biol Ther,* 2007, vol. 6, 432-438
- **LEMOS C ; GIOVANNETTI E ; ZUCALI PA ; AS-SARAF YG ; SCHEFFER GL et al.** Impact of ABCG2 polymorphisms on the clinical outcome and toxicity of gefitinib in non-small-cell lung cancer patients. *Pharmacogenomics,* 2011, vol. 12, 159-170
- **CUSATIS G ; GREGORC V ; LI J ; SPREAFICO A ; INGERSOLL RG et al.** Pharmacogenetics of ABCG2 and adverse reactions to gefitinib. *J Natl Cancer Inst,* 2006, vol. 98, 1739-1742

- **GARCIA-DONAS J ; ESTEBAN E ; LEANDRO-GARCIA LJ ; CASTELLANO DE ; DEL ALBA AG et al.** Single nucleotide polymorphism associations with response and toxic effects in patients with advanced renal-cell carcinoma treated with first-line sunitinib: a multicentre, observational, prospective study. *Lancet Oncol,* 2011, vol. 12, 1143-1150
- **LU JF ; EPPLER SM ; WOLF J ; HAMILTON M ; RAKHIT A et al.** Clinical pharmacokinetics of erlotinib in patients with solid tumors and exposure-safety relationship in patients with non-small cell lung cancer. *Clin Pharmacol Ther,* 2006, vol. 80, 136-145
- **THOMAS F ; ROCHAIX P ; WHITE-KONING M ; HENNEBELLE I ; SARINI J et al.** Population pharmacokinetics of erlotinib and its pharmacokinetic/pharmacodynamic relationships in head and neck squamous cell carcinoma. *Eur J Cancer,* 2009, vol. 45, 2316-2323
- **WHITE-KONING M ; CIVADE E ; GEOERGER B ; THOMAS F ; LE DELEY MC et al.** Population analysis of erlotinib in adults and children reveals pharmacokinetic characteristics as the main factor explaining tolerance particularities in children. *Clin Cancer Res,* 2011, vol. 17, 4862-4871
- **HOUK BE ; BELLO CL ; POLAND B ; ROSEN LS ; DEMETRI GD et al.** Relationship between exposure to sunitinib and efficacy and tolerability endpoints in patients with cancer: results of a pharmacokinetic/pharmacodynamic meta-analysis. *Cancer Chemother Pharmacol,* 2011, vol. 66, 357-371
- *Common Terminology Criteria for Adverse Events VAfhccg,* 09 August 2006
- *Common Terminology Criteria for Adverse Events VAfhccg,* 28 May 2009
- **BLANCHET B ; BILLEMONT B ; CRAMARD J ; BENICHOU AS ; CHHUN S et al.** Validation of an HPLC-UV method for sorafenib determination in human plasma and application to cancer patients in routine clinical practice. *J Pharm Biomed Anal,* 2009, vol. 49, 1109-1114
- **HORNECKER M ; BLANCHET B ; BILLEMONT B ; SASSI H ; ROPERT S et al.** Saturable absorption of sorafenib in patients with solid tumors: a population model. *Invest New Drugs,* 18 October 2011
- **LEVEY AS ; CORESH J ; GREENE T ; MARSH J ; STEVENS LA et al.** Expressing the Modification of Diet in Renal Disease Study equation for estimating glomerular filtration rate with standardized serum creatinine values. *Clin Chem,* 2007, vol. 53, 766-772
- **JANMAHASATIAN S ; DUFFULL SB ; ASH S ; WARD LC ; BYRNE NM et al.** Quantification of lean bodyweight. *Clin Pharmacokinet,* 2005, vol. 44, 1051-1065
- **MORIMOTO M ; NUMATA K ; KONDO M ; HIDAKA H ; TAKADA J et al.** Higher discontinuation and lower survival rates are likely in elderly Japanese patients with advanced hepatocellular carcinoma receiving sorafenib. *Hepatol Res,* 2011, vol. 41, 296-302
- **AZAD NS ; ARAGON-CHING JB ; DAHUT WL ; GUTIERREZ M ; FIGG WD et al.** Hand-foot skin reaction increases with cumulative sorafenib dose and with combination anti-vascular endothelial growth factor therapy. *Clin Cancer Res,* 2009, vol. 15, 1411-1416
- Development and validation of a prediction index for hand-foot skin reaction in cancer patients receiving sorafenib. **DRANITSARIS G ; VINCENT MD ; YU J ; HUANG L ; FANG F et al.** Annals of Oncology. 06 January 2012
- **STRUMBERG D ; CLARK JW ; AWADA A ; MOORE MJ ; RICHLY H et al.** Safety, pharmacokinetics, and preliminary antitumor activity of sorafenib: a review of four phase I trials in patients with advanced refractory solid tumors. *Oncologist,* 2007, vol. 12, 426-437
- **PRADO CM ; LIMA IS ; BARACOS VE ; BIES RR ; MCCARGAR LJ et al.** An exploratory study of body composition as a determinant of epirubicin pharmacokinetics and toxicity. *Cancer Chemother Pharmacol,* 2011, vol. 67, 93-101
- **MATHIJSSEN RH ; SPARREBOOM A.** Influence of lean body weight on anticancer drug clearance. *Clin Pharmacol Ther,* 2009, vol. 85, 23
- **MOURTZAKIS M ; PRADO CM ; LIEFFERS JR ; REIMAN T ; MCCARGAR LJ et al.** A practical and precise approach to quantification of body composition in cancer patients using computed tomography images acquired during routine care. *Appl Physiol Nutr Metab,* 2008, vol. 33, 997-1006
- **GNOTH MJ ; SANDMANN S ; ENGEL K ; RADTKE M.** In vitro to in vivo comparison of the substrate characteristics of sorafenib tosylate toward P-glycoprotein. *Drug Metab Dispos,* 2010, vol. 38, 1341-1346
- **DELBALDO C ; CHATELUT E ; RE M ; DEROUSSENT A ; SERONIE-VIVIEN S et al.** Pharmacokinetic-pharmacodynamic relationships of imatinib and its main metabolite in patients with advanced gastrointestinal stromal tumors. *Clin Cancer Res,* 2006, vol. 12, 6073-6078
- **TREINEN-MOSLEN M ; KANZ MF.** Intestinal tract injury by drugs: Importance of metabolite delivery by yellow bile road. *Pharmacol Ther,* 2006, vol. 112, 649-667
- **GOMO C ; CORIAT R ; FAIVRE L ; MIR O ; ROPERT S et al.** Pharmacokinetic interaction involving sorafenib and the calcium-channel blocker felodipine in a patient with hepatocellular carcinoma. *Invest New Drugs,* 2011, vol. 29, 1511-1514

- **SANCHEZ-FRUCTUOSO et al.** The prevalence of Uridine Diphosphate-Glucuronosyltransferase 1A9 (UGT1A9) Gene Promoter Region Single-Nucleotide Polymorphisms T-275A and C-2152T and its influence on Mycophenolic Acid Pharmacokinetics in Stable Renal Transplant Patients. *Transplantation Proceedings,* 2009, vol. 41, 2313-2316

- **SHIM et al.** Genetic predisposition of hand-foot skin reaction following sorafenib therapy in Korean patients with hepatocellular carcinoma. *J Hepatol,* 2011, vol. 54 (1), 264